(19) Europäisches Patentamt

European Patent Office

Office européen des brevets

(11) **EP 0 514 421 B2**

(12) **NEUE EUROPÄISCHE PATENTSCHRIFT**

(45) Veröffentlichungstag und Bekanntmachung des Hinweises auf die Entscheidung über den Einspruch:
**26.01.2000 Patentblatt 2000/04**

(45) Hinweis auf die Patenterteilung:
**16.08.1995 Patentblatt 1995/33**

(21) Anmeldenummer: 91903318.3

(22) Anmeldetag: **06.02.1991**

(51) Int. Cl.[7]: **A61L 2/00**, C12Q 1/24

(86) Internationale Anmeldenummer:
**PCT/DE91/00099**

(87) Internationale Veröffentlichungsnummer:
**WO 91/12027 (22.08.1991 Gazette 1991/19)**

(54) **VERFAHREN ZUR ABREICHERUNG VON VIREN IN LÖSUNGEN UND ZUR BESTIMMUNG DER ABREICHERUNGSRATE VON VIREN**

PROCESS FOR DEPLETING VIRUSES IN SOLUTIONS AND FOR DETERMINING THE DEPLETION RATE OF THE VIRUSES

PROCEDE D'APPAUVRISSEMENT DE VIRUS CONTENUS DANS DES SOLUTIONS ET DE DETERMINATION DU TAUX D'APPAUVRISSEMENT DE VIRUS

(84) Benannte Vertragsstaaten:
**AT BE CH DE DK ES FR GB GR IT LI LU NL SE**

(30) Priorität: **06.02.1990 DE 4003543**

(43) Veröffentlichungstag der Anmeldung:
**25.11.1992 Patentblatt 1992/48**

(73) Patentinhaber:
**SANORELL PHARMA GmbH & CO.**
**D-72270 Baiersbronn (DE)**

(72) Erfinder: **NADER, Werner, Dr.**
**D-6904 Eppelheim (DE)**

(74) Vertreter:
**Fritzsche, Thomas M., Dr.**
**Fuchs, Mehler, Weiss & Fritzsche**
**Naupliastrasse 110**
**81545 München (DE)**

(56) Entgegenhaltungen:
**EP-A- 0 302 949     EP-A- 0 307 373**

- **Journal of Dairy Science, Band 70, Nr. 10, 1987, American Dairy ScienceAssociation, (Chapaign, Illinois, US), E.A. ZOTTOLA et al.: "Partition oflactic streptococcal bacteriophage during the ultrafiltration concentration ofmilk and whey", Seiten 2013-2021**
- **Biomat., Art. Cells, Art. Org., 16(1-3)(1988), S. 123-128**
- **Vox Sang, Vol. 56 (1989), S. 230-236**
- **Virology, 2nd Ed., Dulbecco and Ginsberg (1988), S.5**

Printed by Xerox (UK) Business Services
2.16.7/3.6

**Beschreibung**

Die Erfindung betrifft ein Verfahren zur Abreicherung von Viren und zur Bestimmung der Abreicherungsrate von Viren in organischem Material.

Arzneimittel, die aus Zellkulturen, Organen oder Blut von Tier oder Mensch gewonnen werden, sind potentiell mit tier- oder humanpathogenen Viren kontaminiert. Bei dem breiten Spektrum von Viren, mit denen die Probe verseucht sein kann, ist es unmöglich, das Ausgangsmaterial auf alle Viren, die vorhanden sein könnten, zu testen. Für einige Virusgruppen gibt es außerdem keine zuverlässige oder genügend sensitive Nachweismethode. Deshalb muß ein Reinigungs- oder Inaktivierungsverfahren durchgeführt werden, durch das pathogene Viren abgereichert werden, so daß auch bei einer massiven Infektion von Ausgangsmaterial oder Zwischenprodukten keine Probleme zu erwarten sind. Das Reinigungsverfahren oder Inaktivierungsverfahren soll dabei so effizient sein, daß Viren um Raten bis zu $10^{12}$ abgereichert werden können.

Um diese Abreicherungsraten zu überprüfen, müssen Proben des Materials mit Viren in extremen Konzentrationsbereichen beaufschlagt (Spiking) und die Abnahme des Virustiters überprüft werden. Da Viren sich in ihrem physikochemischen Verhalten erheblich voneinander unterscheiden können, muß das Material für das Herstellungsverfahren mit einem Spektrum von mindestens vier verschiedenen Virusarten beaufschlagt werden. Dies erfordert einen erheblichen Aufwand, da auch potentiell humanpathogene Viren eingesetzt werden müssen. Diese aufwendigen Verfahren sind beispielsweise in Heimburger,Schwinn, Gratz, Lüben, Kumpe und Herchenhahn, Faktor VIII-Konzentrat, hochgereinigt und in Lösung erhitzt, Arzneimittelforschung 31 (1981), 619-622; Mauler und Hilfenhaus; Inaktivierung von Viren in Faktor VIII-Konzentrat durch Erhitzen in Lösung; Arzneimittelforschung 34 (1984), 1524-1527; Hilfenhaus, Mauler, Friis und Bauer; Safety of human blood products; inactivation of retroviruses by heat treatment at 60°C, Proc. Soc. Exp. Biol. Med., 178 (1985), 580-584, für die Gewinnung von Gerinnungsfaktoren aus Humanserum beschrieben.

Bei der Herstellung von Arzneimitteln wird seit langem zur Entfernung von Bakterien die Sterilfiltration eingesetzt und gilt als sicheres Dekontaminierungsverfahren für diese potentiellen Pathogene. Die Sterilfilter werden dabei vom Hersteller stichprobenweise mit dem kleinsten, außerhalb der Gruppen der Mykoplasmen und L-Formbakterien bekannten Bakterium Pseudomonas diminuta beaufschlagt. Wenn für dieses Bakterium im "Bakterien-Challenge-Test" eine bestimmte Abreicherungsrate bei der Sterilfiltration nachgewiesen werden kann, gilt die Produktionscharge als sicher. Ein derartiges Verfahren ist beschrieben bei Wallhäußer, Praxis der Sterilisation, Thieme Verlag, Stuttgart, 1988, Seiten 324 ff.

Zur Entfernung von Viren können auch Filtrationsverfahren eingesetzt werden, wobei die verwendeten Filter Moleküle und Partikel über 1 Million Dalton zurückhalten müssen. Solche Ultrafilter sind in verschiedensten Ausführungen verfügbar, jedoch handelt es sich - im Gegensatz zu Sterilfiltern - um keine Absolutfilter; d. h. Moleküle und Partikel über 1 Million Dalton werden nicht absolut, sondern nur zu einem großen Teil zurückgehalten. Dabei ist die Rückhalterate nicht nur abhängig vom Filtertyp, sondern sie kann sich sogar von Produktionscharge zur Produktionscharge unterscheiden. Ultrafilter wurden deshalb bisher noch nicht zur Virusentfernung eingesetzt, sondern trugen allenfalls zur Gesamtvirusabreicherung in einem Produktionsverfahren aus mehreren Schritten bei (Werner und Langlius-Gane, Meeting the Regulatory Requirements for Pharmaceutical Production of Recombinant DNA Derived Products, Arzneimittel-Forschung, Bd. 39 (1989), 108-111). Diese Unzuverlässigkeit von Ultrafiltern ist im Produktionsverfahren der Filter begründet. Bei Ultrafiltern, die für einen definierten Molekulargewichtsausschluß vorgesehen sind, können immer größere Mikroporen auftreten, die z. B. für Viren durchlässig sein können. Außerdem können Ultrafilter nicht wie Mikrofilter durch das sogenannte Bubble-Point-Verfahren auf ihre Dichtigkeit getestet werden.

Es war daher Aufgabe der Erfindung, ein Verfahren zur Abreicherung von Viren zur Verfügung zu stellen, mit dem eine Abreicherung um mindestens 12 Zehnerpotenzen erreicht wird. Darüberhinaus war es Aufgabe der Erfindung, ein Verfahren zu schaffen, mit dem die Abreicherungsrate für Viren einfach und präzise bestimmt werden kann und das Auskunft gibt, durch welches Filtrationsverfahren und durch wie viele Filtrationsschritte eine als sicher geltende Abreicherung erreicht wird.

Diese Aufgabe wird gelöst durch ein Verfahren zur Abreicherung von Viren in Lösungen, das dadurch gekennzeichnet ist, daß man die zu reinigende Lösung mittels eines Filters oder einer Filtrationseinheit abreichert, deren Abreicherungsrate vorher ermittelt wurde, indem der Filter oder die Filtrationseinheit mit Viren der Familie Leviviridae beaufschlagt wurde und vor und nach der Filtration der Titer der Viren bestimmt und daraus die Abreicherungsrate ermittelt wurde. Es ist erfindungsgemäß auch möglich andere vergleichbar kleine Bakteriophagen zu verwenden. Diese Bakteriophagen sind vorzugsweise durch einfache Löcher in einem Bakterienrasen nachweisbar.

Erfindungsgemäß ist es möglich, eine Ultrafiltration als einzige Methode zur gesicherten Virusabreicherung ohne zusätzliche Reinigungs- oder Inaktivierungsschritte durchzuführen, indem man im laufenden Prozess die Abreicherungsrate der Viren kontrolliert. Wenn diese Abreicherungsrate vor und nach der Filtration der Produktionscharge bestimmt wird und höher als $10^{12}$ ist, so kann mit größter Sicherheit eine Virus-Kontamination des Produktes ausgeschlossen werden. Bisher wurden Validierungsversuche nur mit tier- oder sogar mit humanpathogenen Viren durchgeführt. Deshalb konnten aus Sicherheitsgründen die validierten Filter nachher nicht mehr verwendet werden. Aus diesem Grund mußten dann neue Filter mit möglicherweise verschiedenen Abreicherungsraten eingesetzt werden. Eine solche Validierung war daher immer nur für einen einzigen Filter gültig und konnte im übrigen auch wegen des damit verbundenen Aufwands nur einmal vor oder nach einer Abreicherung exemplarisch durchgeführt werden. Im Gegensatz dazu ist es erfindungsgemäß möglich im laufenden Verfahren die Virusabreicherung zu verfolgen, also eine In-Prozess-Kontrolle durchzuführen.

Erfindungsgemäß wird die zu reinigende Lösung mittels eines Filters oder einer Filtrationseinheit abgereichert, deren Abreicherungsrate vorher ermittelt wurde. Durch Einsatz von Viren der Gruppe Leviviridae als Testvirus gelingt es, die Abreicherungsrate sicher und ohne größeren Aufwand nachzuweisen.

Die Leviviridae sind mit 23 nm Durchmesser und einem Molekulargewicht von 1,4 Millionen Dalton kleiner als tier- oder humanpathogene Viren (H. Fraenkel-Conrat, The Viruses, Catalogue, Characterization and Classification, Plenum Press, 1982). Sie befallen nur Bestimmte $F^+$-Stämme des harmlosen Darmbakteriums Escherichia coli und werden als RNA-Viren bereits in 10 mM NaOH innerhalb kurzer Zeit hydrolysiert und dabei in ihre molekularen Einzelbestandteile zerlegt. Die kleinsten tier- und/oder humanpathogenen Viren stammen aus der Gruppe der Picornaviren, sind 27 nm im Durchmesser und 2,5 Millionen Dalton schwer. Damit eignen sich die Leviviridae für die Validierung der nach Größe selektionierenden Ultrafilter. Die Filter können danach mit 0,1 M NaOH gewaschen werden, wodurch auch aus Escherichia coli stammende Pyrogene entfernt werden. Die Filter sind dann wieder für die Produktion einsatzbereit. Die für eine In-Prozess-Kontrolle definierten Bedingungen sind damit erfüllt, nämlich durch:

- ein einfaches, innerhalb kurzer Zeit durchführbares und exaktes Validierungsverfahren
- eine Wiederverwendbarkeit der getesteten Filter ohne zusätzliche Verunreinigung des Produkts.

Außerdem können die Leviviridae in extrem hohen Titern von bis zu $10^{14}$ pfu pro ml angezogen werden und durch ein einfaches Plattierungsverfahren selbst in Konzentrationen von 1 pfu pro ml zuverlässig nachgewiesen werden.

Für die Bestimmung wird der Filter mit einer Viruslösung mit einem Titer von über $10^{10}$ pfu/ml beaufschlagt und die Konzentration der Phagen in Filtrat und in der zurückgehaltenen Lösung bestimmt. Die Bestimmung erfolgt in an sich bekannter Weise (z. B. nach der Top-Agar-Methode von N.H. Adams (1959), Bacteriophages, Interscience Publishers, New York). Dazu werden beispielsweise die Phagen mit geeigneten Wirtsbakterien gemischt (z. B. E. coli 3300, ATCC Nr. 19853) und in einer Schicht von 0,6 % Agar-Agar auf Platten mit Nährstoffagar (z. B. 1 % Bactotrypton, 0,5 % Hefeextrakt, 0,5 % NaCl, 0,1 mM $CaCl_2$, 1,5 % Agar-Agar) aufgetragen. Pro Platte sollten $10^7$ bis $10^8$ Bakterien und weniger als 100 Phagen aufgetragen werden. Die Platten werden dann bei 37°C inkubiert, worauf nach 10 Stunden ein Bakterienrasen entsteht. Löcher in diesem Rasen zeigen Virusbefall an und die Anzahl der Löcher ergibt den Virustiter in pfu (plaque-forming-units). Da schon ein Virus einen Plaque hervorrufen kann, können auch mit Standardagarplatten 1 Virus pro ml nachgewiesen werden. Es läßt sich also ein Viruskonzentrationsbereich von über $10^{14}$ bis zu 1 pfu pro ml abdecken.

Durch Bestimmung der Viruskonzentration im Filtrat und in der Lösung vor der Filtration ergibt sich dann die Virusabreicherungsrate. Durch Bestimmung des Virustiter im Konzentrat (d. i. die vor dem Filter zurückgehaltene Lösung) kann errechnet werden, ob Viren durch Absorption an den Filter oder Inaktivierung verloren gingen, was dem Validierungsverfahren eine zusätzliche Sicherheit gibt.

Da sich das Abreicherungsverhalten von Filtrationsmembranen nicht nur von Hersteller zu Hersteller, sondern auch von Produktionscharge zu Produktionscharge erheblich unterscheiden kann, ist es wesentlich, die Abreicherungsrate für Viren für jeden individuellen Filter zu bestimmen.

Bevorzugt überprüft man den für die Reinigung des organischen Materials vorgesehenen Filter bzw. die Filteranlage unter genau definierten Druckbedingungen, die dann beim Reinigungsverfahren später auch eingehalten werden.

Die Filter können dann nach Bestimmung der Abreicherungsrate von den Bakteriophagen und anderen Rückständen wie Pyrogenen durch einfaches Spülen mit Natronlauge entfernt werden und die Filter dann für das Reinigungsverfahren des organischen Materials eingesetzt werden. Dies ist ein weiterer Vorteil des erfindungsgemäßen Verfahrens, da dies bei Verwendung von tier- oder humanpathogenen Viren zur Bestimmung der Abreicherungsrate wegen der großen Gefahr der Kontamination mit derartigen Viren nicht möglich wäre.

Bevorzugt werden aus der Gruppe der Leviviridae für das erfindungsgemäße Verfahren die Viren MS2, f2, f4, Qβ, Vk, ST oder R17 oder vergleichbare Stämme eingesetzt, die bei H. Fraenkel, Conrat, The Viruses-Catalogue, Characterization and Classification, Plenum Press, New York, 1982, beschrieben sind. Besonders bevorzugt wird der Bakteriophage fr als Testvirus verwendet, der bei ATCC unter der Nummer 15767-B1 hinterlegt ist und in Knolle und Hoffmann-Berling, Virology, Band 123, 271-273 (1964) beschrieben ist. Dieser Phage besteht aus einem runden Protein-RNA-Komplex in Form eines Polyeders mit einem Durchmesser von 23 nm und hat ein Molekulargewicht von 1,4 Millionen Dalton.

In einer bevorzugten Ausführungsform wird das organische Material durch Ultrafiltration in Spiralpatronen gereinigt. Die Patronen werden dabei über eine Pumpe beschickt. Vor Einsatz der Filtrationspatronen wird der Virus-Abreicherungsfaktor mit dem Testvirus ermittelt. Die Testlösung, die die Testviren in bekannter Menge enthält, wird über die Patrone im Tangentialfluß filtriert unter genau definierten Bedingungen hinsichtlich des Drucks. Im Filtrat wird dann nach bekannten Verfahren der Virustiter bestimmt. Anschließend wird dann das organische Material in derselben Patrone unter Einhaltung der gleichen Bedingungen gereinigt.

Die Testviren können vor Einsatz für das erfindungsgemäße Verfahren in an sich bekannter Weise bis auf einen Titer von $10^{14}$ angezogen werden. Als Wirtsbakterium für die Bakteriophagen eignet sich z. B. Escherichia coli 3300, ATCC Nr. 19853. Kulturmedien zur Anzucht von Phagen sind bekannt. Geeignet ist beispielsweise das von Luria und Bertani beschriebene Medium, das auf 1 l destilliertes Wasser 10 g Bactotrypton, 5 g Hefeextrakt und 5 g NaCl enthält und einen pH von 7,5 hat, der gegebenenfalls mit NaOH eingestellt wird. Zur Gewinnung der Viren werden diese aus dem Kulturmedium ausgefällt durch Zugabe eines Fällungsmittels, beispielsweise Polyethylenglykol PEG6000. Die Viren werden dann in Puffer resuspendiert und in dem Puffer auf den gewünschten Titer eingestellt. Als Puffer ist beispielsweise ein Tris-HCl-Puffer mit pH 7,5 geeignet, der 100 mM NaCl under 3 mM $CaCl_2$ enthält. Die Bestimmung des Titers kann erfolgen nach der Top-Agar-Methode. Anschließend wird diese eingestellte Phagensuspension dem für das organische Material gewünschten Filtrationsverfahren unterworfen. Nach Abschluß dieser Filtration wird der Virustiter bestimmt und aus der Abnahme des Titers kann der Abreicherungsfaktor berechnet werden. Der Virustiter wird in der Einheit "pfu" (Plaque-forming-units) angegeben und bedeutet die Anzahl von Löchern im Bakterienrasen, die durch die Virusinfektion hervorgerufen werden. Die Filtration wird dann nach Spülen der Patrone mit Natronlauge und Neutralisieren durch Spülen mit destilliertem Wasser so lange wiederholt, bis die gewünschte Abreicherungsrate erreicht ist. Ebenso können auch mehrere Filter hintereinander geschaltet werden, über die die Filtration hintereinander durchgeführt wird. Nach der Bestimmung des Abreicherungsfaktors werden die durch die Phagen eingetragenen Pyrogene durch Spülen der Patrone mit Natronlauge entfernt und sind dann nach Neutralisieren für den Produktionsprozeß einsatzfähig.

Es hat sich überraschenderweise gezeigt, daß das erfindungsgemäße Verfahren besonders geeignet ist, die Virusabreicherung während des Produktionsprozesses bei der Herstellung von keimfreien Extrakten aus biologischem Material als sogenannte In-Prozess-Kontrolle zu verwenden. Es wurde nämlich gefunden, daß die Abreicherung von Markerstoffen in der zu reinigenden Probe mit der Abreicherungsrate der Viren korreliert. Auf diese Weise ist es möglich, durch Bestimmung der Abreicherung des Markerstoffes die Virusabreicherung zu verfolgen.

Dabei wird erfindungsgemäß so vorgegangen, daß man die Abreicherungsrate von Virus und Marker bestimmt, das Verhältnis aus den beiden Abreicherungsraten bildet, d.h., daß man daraus eine Eichkurve erstellt und im laufenden Prozess durch Bestimmung der Markerabreicherung anhand der Eichkurve die Virusabnahme verfolgt.

Als Markerstoffe werden üblicherweise leicht bestimmbare Substanzen verwendet, die vorzugsweise bereits im zu reinigenden System vorhanden sind. Es ist jedoch auch möglich, solche Markerstoffe dem abzureichernden System zuzusetzen. Bevorzugte Markerstoffe sind Proteine, Peptide und/oder Nukleinsäuren. Es ist jedoch auch möglich, synthetische Substanzen, insbesondere Oligo- und Polymere als Markerstoffe einzusetzen. Solche Polymere sind dem Fachmann bekannt und können durch leichte, einfache Versuche für das jeweilige System gefunden werden. Als besonders bevorzugter Marker wird erfindungsgemäß BSA verwendet.

Bevorzugte zu reinigende Präparationen sind üblicherweise biologische Materialien, insbesondere solche aus pflanzlichen und tierischen Organismen. Vorzugsweise werden solche Präparationen aus Organen, Geweben und/oder Zellen gewonnen. Bevorzugte Organe sind, Milz, Thymus und/oder Knochenmark. Das erfindungsgemäße Verfahren eignet sich jedoch ebenfalls zur Reinigung von biologischem Material, das aus Körperflüssigkeiten oder auch aus bakteriellem oder virellem Material, insbesondere aus pathogenem Material gewonnen wurde.

In einer bevorzugten erfindungsgemäßen Ausfürungsform wird der Abreicherungsfaktor mit vier verschiedenen Viren bestimmt.

Die Erfindung wird durch die Figuren und die folgenden Beispiele erläutert.

Fig. 1     zeigt das Schema eines Filtrationssystems, das unter dem Namen Amicon S1 im Handel ist.
Fig. 2     zeigt eine Filtrationspatrone des Filtrationssystems von Fig. 1.

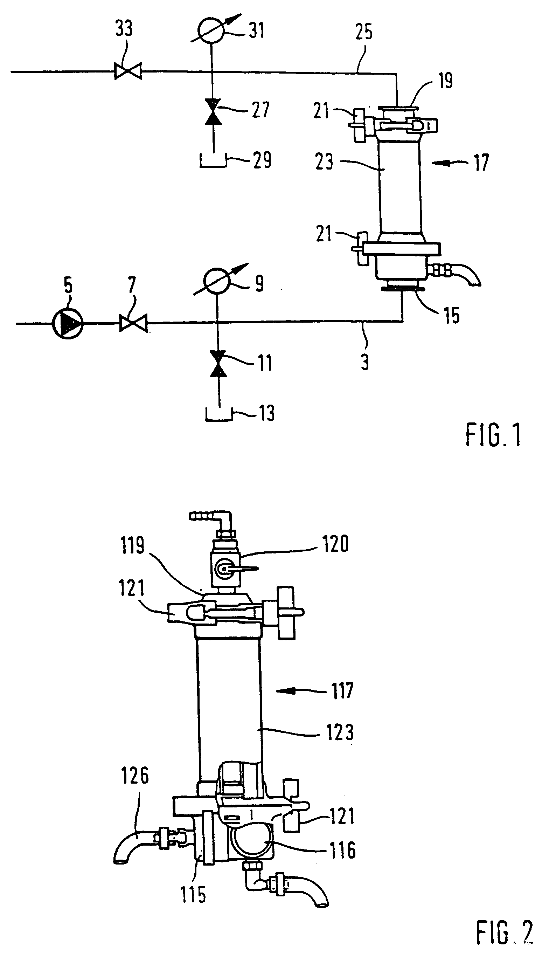

Figur 1 zeigt ein Filtrationssystem zur Ultrafiltration von organischen Lösungen. Aus einem Vorratstank (nicht gezeigt) wird die Lösung über eine Leitung 3, die mit einer Umlaufpumpe 5 versehen ist und ein Drosselventil 7, einen Druckmesser 9, ein Absperrventil 11 und eine Abflußöffnung 13 aufweist, über den Einlaßkopf 15 in die Filtrationsanordnung 17 geleitet. Die Filtrationsanordnung 17, die mit einem Einlaßkopf 15 und einem Auslaßkopf 19 versehen ist und Klemmen 21 aufweist, enthält eine Spiralpatrone 23. Aus der Filtrationsanordnung 17 wird das Filtrat über eine Leitung 25, die ein Absperrventil 27, eine Abflußöffnung 29, einen Druckmesser 31 und ein Rückdruckventil 33 aufweist, in einen weiteren Vorratsbehälter (nicht gezeigt) geführt.

Figur 2 zeigt die Filtrationsanordnung 117. Die Filtrationsanordnung 117 weist einen Einlaßkopf 115 auf, der mit einem Druckmesser 116 und einer Klemmvorrichtung 121 versehen ist. In den Einlaßkopf führt ein Permeat Port 126. Die Filtrationsanordnung 117 enthält eine Spiralpatrone 123. Am oberen Teil der Filtrationsanordnung 117 befindet sich der Auslaßkopf 119, der mit einem Rückdruckventil 120 und einer Klemmvorrichtung 121 versehen ist.

**Beispiel 1**

Test einer Sartorius-Polysulfonmembran mit einem molekularen Ausschluß von 100.000 Dalton auf Virusabreicherung

Eine Filtrationskassette aus Polysulfon der Firma Sartorius (Göttingen, BRD) wurde mit einer Phagensuspension im Tangentialfluß beaufschlagt, wobei die im Prototypverfahren beschriebenen Bedingungen eingehalten wurden. Tabelle 1 zeigt das Ergebnis dieser Probeläufe bei drei verschiedenen Partialdrücken. Der Phage wurde bei allen Partialdrücken nur um eine Zehnerpotenz abgereichert. Um eine Abreicherung um den Faktor $10^{10}$ zu erreichen, müßte also die Filtration mindestens 10mal wiederholt und diese Wiederholung durch Beaufschlagung mit dem Phagen fr kontrolliert werden.

**Beispiel 2**

Für dieses Beispiel wurde die Virusabreicherung durch das Filtrationssystem Amicon S1 mit einer Ultrafiltrationsmembran mit einem molekularen Ausschluß von 30.000 Dalton getestet.
Das technische Prinzip des Filtrationssystems ist in Figur 1 dargestellt, die Filtrationspatrone ist in Figur 2 gezeigt. Die zu filternde Lösung strömt dabei im Tangentialfluß über die Membran und ein Teil der Lösung wird durch den über die Membran bestehenden Transmembrandruck ($P_t$) abfiltriert. Der Druck am Eingang 15 ($P_a$) und am Ausgang 19 ($P_b$) des Geräts kann an zwei Manometern abgelesen werden. Der sich über die Membran ausbildende Transmissionsdruck wird nach der Formel

$$P_t = \frac{P_a + P_b}{2} - P_p$$

berechnet, wobei $P_p$ der Permeatdruck bedeutet, der üblicherweise gleich Null und $\neq P_a$ ist. Die Proben wurden mit einer Schlauchquetschpumpe 31 bei 130 Umdrehungen pro Minute und einem Schlauchinnendurchmesser von 8 mm in die Patrone 23 gepumpt und der Transmissionsdruck durch Regulierung des Auslaßventils auf 0,2, 0,4 und 0,7 bar eingestellt.
Zum Testen der Patrone wurden Phagenlösungen mit einem Titer von 7,8 x $10^9$ pfu (plaque forming units) pro ml (in 10 mM Tris-Cl Puffer, ph 7,5 mit 100 mM NaCl und 1 mg Rinderserumalbumin pro ml) über die Membran gepumpt. Pro Test wurden je 1,5 l der Phagenlösung filtriert. Zwischen jeder Filtration wurde die Patrone mit 0,1 M NaOH gewaschen und dann solange mit PBS (phosphatgepufferte Kochsalzlösung) gespült, bis das Eluat neutralisiert war. Durch diesen Waschvorgang wurden im System verbleibende Phagen vollständig inaktiviert. Gelagert wurde die Patrone in 10 mM NaOH.
Tabelle 2 enthält die Ergebnisse dieses Tests mit der Filtrationspatrone S1Y30 der Seriennummer 8864. Es ergaben sich Abreicherungen um 4,53 Zehnerlogarithmen unmittelbar nach Inbetriebnahme und um 4,4 Zehnerlogarithmen nach mehrmonatiger Lagerung der Patrone in 10 mM NaOH nach der ersten Filtration.

**Beispiel 3**

Bestimmung der Abreicherungsrate in einer Ultrafiltrationsanlage mit dem Bakteriophagen fr

Überprüft wurde eine Spiralpatrone S1Y30 der Firma Amicon mit der Serien-Nr. 8864. Hierzu wurde je 600 ml einer Phagensuspension mit einem Ausgangstiter von 3x$10^{10}$ in fünf parallelen Ansätzen dreimal hintereinander über die Patrone gefiltert. Zwischen jedem Filtrationsschritt wurde die Patrone mit 2 l RO-Wasser (über Reverse Osmose hochgereinigtes Wasser) gespült. Aus Tabelle 3 geht hervor, daß in allen fünf Parallelansätzen nach drei Filtrationen über die Patrone kein infektiöser Phage fr in 1 ml des Permeats enthalten ist.

**Beispiel 4**

Abreicherung der Testviren um den Faktor 12

Getestet wurde die Filtrationspatrone S1Y30 mit der Seriennummer 10330. Die Phagenlösung bestand aus 600 ml Phagenpuffer (Methoden) mit 600 mg Rinderserumalbumin und 50 ml Phagenkonzentrat (Titer: $1,3 \times 10^{12}$ pfu (plaque forming units)/ml). Die Filtration wurde zunächst dreimal hintereinander durchgeführt. Das Volumen des Diltrats verringert sich von 600 über 400 auf 380 ml. Die Filtrationszeiten betrugen je Schritt ca. 20 Minuten. Zwischen jeder Filtration wurde die Patrone zur Inaktivierung von Phagenrückständen mit 1 l 10 mM Natronlauge gewaschen und dann mit dest. Wasser bis zur Neutralität (gemessen mit der pH-Elektrode) gespült.
Der Virusgehalt in den einzelnen Filtraten wurde bestimmt und ist in Tabelle 4 dargestellt.
Da bereits nach der 2. Filtration in 1 ml Filtrat kein Bakteriophage fr mehr nachweisbar ist, wurde das letzte Filtrat (380 ml) erneut mit 40 ml Viruskonzentrat (Titer: $5,2 \times 10^{12}$ pfu/ml) angereichert und dreimal hintereinander filtriert. Wie aus Tabelle 1 ersichtlich, lassen sich in 1 ml des Filtrats nach der zweiten Filtration keine Phagen mehr nachweisen.
Aus den Daten der Virusabreicherung (Tabelle 4) geht hervor, daß mit der eingesetzten Ultrafiltrationspatrone nach der ersten Filtration der Virustiter um 6,92 und 7,22 Zehnerlogarithmen abnahm. Nach der jeweils zweiten Filtration waren bereits keine Phagen mehr im Filtrat nachweisbar. Demnach wurden die Viren nach den ersten zwei Filtrationen um insgesamt 11 Zehnerpotenzen und nach den weiteren zwei Filtrationen um 11,7 Zehnerpotenzen reduziert, woraus sich rechnerisch eine Gesamtreduktion um 22,7 Zehnerpotenzen nach vier Filtrationen ergibt. Die in der Literatur häufig empfohlene Abreicherung um 12 bis 16 wird mit 18,22 Zehnerpotenzen bereits nach drei Filtrationen überschritten. Die Virusabreicherung ist bei dieser Produktionsserie mit 7 Zehnerlogarithmen besser als bei der in den Beispielen 2 und 3 verwendeten Produktionsserie mit ca. 4,5 Zehnerlogarithmen (Tabelle 3 und 4). Zwischen den einzelnen Produktionschargen können also erhebliche Unterschiede in der Virusabreicherung festgestellt werden, was wiederum die Notwendigkeit einer sorgfältigen Validierung mit einem Testviren unterstreicht.

**Beispiel 5**

Kontrolle der Virusabreicherung eines Thymusextraktes durch Bestimmung von BSA

Kälbern wurde die Thymusdrüse entnommen und homogenisiert. Aus diesem Homogenisat wurde auf an sich bekannte Weise ein Extrakt gewonnen. Dem so erhaltenen Extrakt wurde als Testvirus der Bakteriophage fr (ATCC Nr. 15767-B1; Knolle und Hoffmann Verlag, Virology, Bd. 123, 271-273 (1964) zugesetzt und der Gehalt an BSA sowie an Purin- und Pyrimidinbasen wurde mittels einer HPLC-Analyse auf eine übliche, dem Fachmann bekannte Weise bestimmt.
Die mit dem Testphagen versetzte Probe wurde dann, wie in den Beispielen 3 und 4 beschrieben, über eine Filtrationspatrone S1Y30 mit der Seriennummer 10330 (Amicon Div.; W. R. Grace & Co.; Danvers, M. A., U.S.A.) filtriert und sowohl die Virusabreicherung als auch die Abnahme an BSA bestimmt. Die Abnahme des Virus korrellierte mit der Abnahme an BSA.
Der Bakteriophage fr ( ATCC 15767-B1 ) wurde am 19. November 1964 bei der American Type Culture Collection, 12301 Parklawn Drive, Rockville, Maryland 20852-1776 USA hinterlegt, und ist seit dem 19. November frei erhältlich.
E. Coli 3300 (ATCC 19853) wurde am 12. Januar 1967 bei der American Type Culture Collection, 12301 Parklawn Drive, Rockville, Maryland 20852-1776 USA hinterlegt, und ist seit dem 12 Januar 1967 frei erhältlich.

T a b e l l e   1

Ultrafiltration mit dem Sartorius-Tangentialflußsystem
Ultrafilter: Polysulfonmembran mit einem molekularen
Cut off von 100.000 Dalton
Modultyp: 303 145 69 01 W
Versuchsbedingungen:

1. Lauf: Permeatfluß - 4 ml/sec, Partialdruck - 0,8 bar
Volumen des Eluats: 660 ml, Zeitdauer der Filtration: 2 min 31 sec,
Volumen des Konzentrats: 66 ml

2. Lauf: Permeatfluß - 2,3 ml/sec, Partialdruck - 0,4 bar
Volumen des Eluats und des Konzentrats wie oben, Zeitdauer der
Filtration: 4 min 32 sec

3. Lauf: Permeatfluß - 5 ml/sec, Partialdruck - 1,6 bar
Volumen des Permeats und des Konzentrats wie oben, Zeitdauer der
Filtration: 1 min 56 sec

Verteilung der Bakteriophagen:

| Lauf Nr. | Ausgangs- lösung pfu/ml | Konzentrat pfu/ml | Permeat pfu/ml | Phageneliminierungsrate (log10 pfu 1 / pfu 2) |
|---|---|---|---|---|
| 1 | 4,30E+06 | 5,00E+06 | 1,80E+05 | 1,378196 |
| 2 | 4,30E+06 | 6,35E+06 | 1,50E+05 | 1,4573772 |
| 3 | 1,70E+09 | 7,40E+09 | 1,60E+08 | 1,0263289 |

EP 0 514 421 B2

Tabelle 2

| Test des Ultrafiltrationssystems Amicon S1Y30 auf Durchlässigkeit für den Testphagen fr | | | | | | |
|---|---|---|---|---|---|---|
| Je 1,5 ml einer Phagentestlösung (Bakteriophage fr in 10 mM Tris-Cl, pH 7,5, 100 mM NaCl und 1 mg Rinderserumalbumin pro ml) wurden bei den angegebenen Transmissionsdrücken über die Spiralpatrone filtriert. | | | | | | |
| | 1. Filtration*) | | | 2. Filtration*) | | |
| Transmissions-druck | Lösung der Fil-tration | Permeat | Konzentrat (10 % der Aus-gangslösung) | Lösung vor Fil-tration | Permeat | Konzentrat (10 % der Aus-gangslösung) |
| 3 psi | $7,8 \times 10^9$ | $2,3 \times 10^5$ | $1,3 \times 10^{10}$ | $2,3 \times 10^5$ | 0 | $9,6 \times 10^5$ |
| 6 psi | $7,8 \times 10^9$ | $6,9 \times 10^5$ | $6,4 \times 10^9$ | $6,9 \times 10^5$ | $1,5 \times 10^1$ | $2,8 \times 10^6$ |
| 10 psi | $7,8 \times 10^9$ | $7,3 \times 10^5$ | $1,07 \times 10^{10}$ | $7,3 \times 10^5$ | $1,5 \times 10^1$ | $3,3 \times 10^6$ |

*) Der Phagentiter ist in plaque forming units (pfu) pro ml angegeben.

Tabelle 3

| Validierung der Ultrafiltrationspatrone S1X30 mit dem Testphagen fr [a] Titer des Testphagen fr in Plaque Forming Units [b] pro Milliliter (pfu/ml) | | | | |
|---|---|---|---|---|
| | In der Ausgangslösung | Im Permeat der 1. Filtration | Im Permeat der 2. Filtration | Im Permeat der 3. Filtration |
| 1. Testlauf | $3 \times 10^{10}$ | $3,4 \times 10^6$ | $2,9 \times 10^2$ | 0 |
| 2. Testlauf | $3 \times 10^{10}$ | $4,5 \times 10^6$ | $7,3 \times 10^1$ | 0 |
| 3. Testlauf | $3 \times 10^{10}$ | $3,9 \times 10^6$ | $1,1 \times 10^2$ | 0 |
| 4. Testlauf | $3 \times 10^{10}$ | $7,0 \times 10^6$ | $7,0 \times 10^1$ | 0 |
| 5. Testlauf | $3 \times 10^{10}$ | $5,4 \times 10^6$ | $7,6 \times 10^1$ | 0 |
| Mittelwerte | | $4,84 \times 10^6$ | $1,24 \times 10^2$ | |
| Standardabweichungen | | $1,2 \times 10^6$ | $8,4 \times 10^1$ | |

[a] Je 600 ml der Phagentestlösung (Bakteriophage fr in Phagenpuffer: 10 mM Tris-Cl, 10 mM CaCl$_2$, 0,15 M NaCl und 1 mg Rinderserumalbumin pro ml) wurden dreimal hintereinander bei einem Transmissionsdruck von 3 psi über die Amicon Ultrafiltrationspatrone S1Y30 filtriert.

[b] Der Titer des Phagen wurde mit Escherichia coli Stamm 3300 auf Agarplatten nach dem bei Davis und Sinsheimer ("TOP-AGAR-METHODE", J. Mol. Bio., 1963, Bd. 2, Seiten 203-207) beschriebenen Verfahren bestimmt, wobei die Permeate in dem Phagenpuffer (siehe [a]) verdünnt wurden. In Abweichung zur Originalvorschrift wurde auf Luria-Bertani Nährboden titriert (10 g Trypton, 5 g Hefeextrakt, 5 g NaCl auf 1 l RO-Wasser, pH auf 7,5 mit NaOH eingestellt). Dem "Top-Agar" wurde außerdem 3 mM CaCl$_2$ zugesetzt.

Tabelle 4

| Virustiter in den Filtraten der einzelnen Filtrationsschritte mit der Ultrafiltrationspatrone Amicon S1Y30 | |
|---|---|
| Filtrationsschritt | Virustiter (pfu/ml) |
| Ausgangslösung | $1 \times 10^{11}$ |

Tabelle 4 (fortgesetzt)

| Virustiter in den Filtraten der einzelnen Filtrationsschritte mit der Ultrafiltrationspatrone Amicon S1Y30 | |
|---|---|
| Filtrationsschritt | Virustiter (pfu/ml) |
| 1. Filtration | $1,2 \times 10^4$ |
| 2. Filtration | 0 |
| 3. Filtration | 0 |
| Zugabe neuer Testphagen | $5 \times 10^{11}$ |
| 1. Filtration | $3 \times 10^4$ |
| 2. Filtration | 0 |
| 3. Filtration | 0 |

**Patentansprüche**

1. Verfahren zur Abreicherung von Viren und/oder pathogenem Material in organischen Präparationen, **dadurch gekennzeichnet**, daß man das zu reinigende Material mittels eines Ultrafilters oder einer Ultrafiltrationseinheit abreichert, deren Abreicherungsrate vorher ermittelt wurde, indem der Filter oder die Filtrationseinheit mit Viren der Familie Leviviridae oder anderer vergleichbar kleiner Bakteriophagen beaufschlagt wurde und vor und nach der Filtration der Titer der Viren bestimmt und daraus die Abreicherungsrate ermittelt wurde.

2. Verfahren nach Anspruch 1, **dadurch gekennzeichnet**, daß man als Testvirus MS2, f2, f4, Qβ, Vk, ST oder R17 verwendet.

3. Verfahren nach einem der Ansprüche 1, **dadurch gekennzeichnet**, daß man als Testvirus den Bakteriophagen fr, ATCC Nr. 15767-B1 verwendet.

4. Verfahren nach einem der vorhergehenden Ansprüche, **dadurch gekennzeichnet**, daß man die den Testvirus enthaltende Probe unter Einhaltung genau definierter Druckbedingungen ultrafiltriert und zur Aufreinigung des organischen Materials dieselben genau definierten Bedingungen einhält.

5. Verfahren zur Bestimmung der Abreicherungsrate von Viren und/oder pathogenem Material in organischen Präparationen, **dadurch gekennzeichnet**, daß man eine Probe des Materials mit Viren der Gruppe Leviviridae als Testvirus versetzt, diese Probe den für das Material vorgesehenen Ultrafiltrationschritten unterzieht und vor und nach Ultrafiltration die Anzahl der Viren bestimmt und daraus die Abnahme des Virustiters feststellt.

6. Verfahren nach einem der vorhergehenden Ansprüche, **dadurch gekennzeichnet**, daß man die Abreicherungsrate eines im zu reinigenden Material bereits enthaltenen oder zugesetzten Markerstoffs bestimmt, das Verhältnis von den Abreicherungsraten des Markers und des Virus bildet und die Virusabreicherung durch die Abreicherung des Markerstoffes verfolgt.

7. Verfahren nach einem der vorhergehenden Ansprüche, **dadurch gekennzeichnet**, daß das zu reinigende Material aus Pflanzenteilen oder aus menschlichen oder tierischen Geweben oder Organen oder aus Bakterien- oder Virusextrakten gewonnen wird.

8. Verfahren nach einem der vorhergehenden Ansprüche, **dadurch gekennzeichnet**,

daß das zu reinigende Material aus Milz, Thymus und/oder Knochenmark gewonnen wird.

9. Verfahren nach einem der Ansprüche 6 bis 8,
**dadurch gekennzeichnet**,
daß man als Markerstoff ein Protein oder eine Nukleinsäure verwendet.

10. Verfahren nach einem der Ansprüche 6 bis 9,
**dadurch gekennzeichnet**,
daß man als Markerstoff BSA verwendet.

**Claims**

1. Method of removing viruses and/or pathogenic material from organic preparations, characterised in that the material to be purified is removed by means of an ultrafilter or an ultrafiltration unit, the removal rate of which has previously been ascertained by admitting into the filter or filtration unit viruses of the leviviridae family or other comparatively small bacteriophages, by determining the titer of the viruses before and after the filtration, and by ascertaining the removal rate therefrom.

2. Method according to claim 1, characterised in that MS2, f2, f4, Qβ, Vk, ST or R17 is used as the test virus.

3. Method according to claim 1, characterised in that the bacteriophage fr, ATCC No. 15767-B1, is used as the test virus.

4. Method according to one of the preceding claims, characterised in that the specimen containing the test virus is ultrafiltered, whilst adhering to precisely defined pressure conditions, and the same precisely defined conditions are adhered to for the purification of the organic material.

5. Method of determining the removal rate of viruses and/or pathogenic material in organic preparations, characterised in that a specimen of the material is mixed with viruses of the leviviridae group as the test virus, this specimen is subjected to the ultrafiltration steps intended for the material, the number of viruses is determined before and after ultrafiltration, and the decrease in the virus titer is detected.

6. Method according to one of the preceding claims, characterised in that the removal rate of a marker substance is determined, which substance was already contained in or subsequently added to the material to be purified, the ratio of the removal rates between the marker and the virus is formed, and the removal of the virus is tracked by the removal of the marker substance.

7. Method according to one of the preceding claims, characterised in that the material to be purified is obtained from plant parts, from human or animal tissues or organs, or from bacteria or virus extracts.

8. Method according to one of the preceding claims, characterised in that the material to be purified is obtained from spleen, thymus and/or bone marrow.

9. Method according to one of claims 6 to 8, characterised in that a protein or a nucleic acid is used as the marker substance.

10. Method according to one of claims 6 to 9, characterised in that BSA is used as the marker substance.

**Revendications**

1. Procédé d'appauvrissement de préparations organiques en virus et/ou matrice pathogène, caractérisé en ce que l'on appauvrit la substance à purifier au moyen d'un ultrafiltre ou d'une unité d'ultrafiltration dont on a établi préalablement le taux d'appauvrissement en chargeant le filtre ou l'unité de filtration de virus de la famille des Leviviridae ou d'autres bactériophages de taille comparable, en déterminant le titre de virus avant et après la filtration et en établissant ainsi le taux d'appauvrissement.

2. Procédé selon la revendication 1, caractérisé en ce qu'on utilise comme virus tests MS2, f2, f4, Qβ, Vk, ST ou R17.

3. Procédé selon la revendication 1, caractérisé en ce qu'on utilise comme virus test le bactériophage fr, ATCC n° 15767-B1.

4. Procédé selon une des revendications précédentes, caractérisé en ce qu'on procède à l'ultrafiltration de l'échantillon contenant le virus test en respectant des conditions de pression exactement définies et qu'on respecte ces mêmes condition exactement définies pour la purification de la substance organique.

5. Procédé de détermination du taux d'appauvrissement de préparations organiques en virus et/ou substance pathogène, caractérisé en ce qu'on ajoute à un échantillon de substance des virus du groupe Leviviridae en tant que virus tests, qu'on soumet cet échantillon aux étapes de purification prévues pour la substance et qu'on détermine le nombre de virus avant et après l'ultrafiltration et qu'on constate ainsi la diminution du titre du virus.

6. Procédé selon une des revendications précédentes, caractérisé en ce qu'on détermine le taux d'appauvrissement d'une substance marqueur déjà contenue dans la substance à purifier ou ajoutée, qu'on forme le rapport des taux d'appauvrissement du marqueur et du virus et qu'on suit l'appauvrissement en virus par l'appauvrissement en marqueur.

7. Procédé selon une des revendications précédentes, caractérisé en ce que la substance à purifier provient de fragments végétaux ou de tissus ou organes humains ou animaux ou d'extraits de bactéries ou de virus.

8. Procédé selon une des revendications précédentes, caractérisé en ce que la substance à purifier provient de rate, de thymus et/ou de moelle osseuse.

9. Procédé selon une des revendications 6 à 8, caractérisé en ce qu'on utilise comme marqueur une protéine ou un acide nucléique.

10. Procédé selon une des revendications 6 à 9, caractérisé en ce qu'on utilise de la BSA comme marqueur.

FIG.1

FIG.2